# EUROPEAN PATENT SPECIFICATION

(11) **EP 1 446 160 B1**
(45) Date of publication and mention of the grant of the patent: **02.04.2008**
(21) Application number: 02777584.0
(22) Date of filing: 31.10.2002
(51) Int. Cl.: A61K 47/02, A61K 31/616

(54) **ASPIRIN-CONTAINING TRANSDERMAL PHARMACEUTICAL COMPOSITION FOR THE TREATMENT OF CALCIFICATION**
ASPIRIN ENTHALTENDE TRANSDERMALE PHARMAZEUTISCHE ZUSAMMENSETZUNG ZUR BEHANDLUNG VON VERKALKUNG
COMPOSITION PHARMACEUTIQUE TRANSDERMIQUE CONTENANT DE L'ASPIRINE DESTINEE AU TRAITEMENT DE LA CALCIFICATION

(30) Priority: 16.11.2001 HU 0104968
(43) Date of publication of application: 18.08.2004
(73) Proprietor: Dardai, Zoltan, 1157 Budapest (HU)
(72) Inventor: Dardai, Zoltan, 1157 Budapest (HU)
(74) Representative: Beszédes, Stephan G.
(86) International application number: PCT/HU2002/000112
(87) International publication number: WO 2003/041743

(56) References cited:
- WO-A-01/03774
- US-A- 4 003 989
- RAINSFORD K D: "Electronmicroscopic observations on the effects of orally administered aspirin and aspirin bicarbonate mixtures on the development of gastric mucosal damage in the rat" GUT, vol. 16, no. 7, 1975, pages 514-527, XP008014067 ISSN: 0017-5749

## Description

The invention is concerned with the use of acetylsalicylic acid for preparing a transdermal pharmaceutical composition for the treatment of calcification.

Calcification of cartilages, joints and interior organs is a disorder impending over everyone with the progress in lifetime; said disorder plays a causative role in the development of numerous diseases or medical conditions (such as joint inflammations, rheuma, locomotor diseases and deformations). Numerous therapeutical methods have been known for the treatment of calcification and its consequences. These methods include non-drug treatments (such as diet, curative gymnastics, massage, mud pack and balneotherapy) and various drug therapies (such as administration of antiphlogistic, analgesic and muscle-relaxant agents), which are frequently used in combination. An important representative of analgesic and antiphlogistic agents is acetylsalicylic acid (aspirin^{®}), which is used primarily for the treatment of rheumatic arthritis, rheumatoid arthritis and arthritis-like conditions, usually in the form of orally administerable compositions or injections, but sometimes as a transdermal composition, too [Byoin Yakugaku 17(5), 335-340 (1991) (see C.A. 116, 158740q); Drugs of Today 33(5), 299-306(1997); US patents Nos. 4,275,059, 5,260,066 and 5,612,382; GB patent No. 1,036,314; French patent No. 2,295,753]. Some of the papers listed above, furthermore Arch. Int. Pharmacodyn. 177(1), 211-223(1969) also indicate that acetylsalicylic acid possesses anti-calcification effects.

However, it is well known that acetylsalicylic acid once entered the organism metabolises within a short period of time and decomposes to salicylic acid and acetic acid. Results of the tests performed by the Applicant have fully supported this quick metabolism. Although the two metabolites are also effective in reducing established calcifications with the formation of calcium salicylate and calcium acetate [this effect of salicylic acid has been discussed in Arch. Int. Pharmacodyn. 177(1), 211-223(1969) cited above], the appearance of the formed two compounds, particularly of calcium acetate, in the organism is highly objectionable from nephrological aspects, due to its specific crystal structure. Presumably this explains why acetylsalicylic acid has not been utilized in practice to cease already established calcification or to reduce its extent, despite of its promising activity. WO 01/03774 discloses a topical composition comprising acetylsalicylic acid for the treatment of calcification.

From Rainsford KD (1975) Gut 16: 514-527 there has been known an aqueous solution comprising an equimolar amount of acetylsalicylic acid and sodium bicarbonate, pH 5.6. No mention of the use for the treatment of calcification nor of an influence of the sodium bicarbonate on the metabolism of acetylsalicylic acid has been made. In US 4,003,989 A mixtures comprising acetylsalicylic acid (ASA) and either potassium bicarbonate and carbonate, or sodium bicarbonate and carbonate has been described. The mixtures are dissolved in water to obtain a drink.

Now it has been found that in a medium buffered to pH 5.5 to 7 and in the presence of an alkali metal or ammonium carbonate or hydrocarbonate the metabolism of acetylsalicylic acid can be slowed down to such an extent that at the area of calcification the conversion of acetylsalicylic acid to calcium acetylsalicylate proceeds much faster than its metabolism to salicylic acid and acetic acid. Acetylsalicylic acid which has been once bound in the form of calcium acetylsalicylate does not metabolise further but is excreted from the organism in unchanged form without causing nephrological damages.

This recognition enables one to produce a nephrologically harmless pharmaceutical composition suitable for the targeted treatment of calcification.

Thus the subject matter of the invention is the use of acetylsalicylic acid together with a carrier, diluent and/or other auxiliary agent suitable for transdermal application, and for 1 g of acetylsalicylic acid an alkali metal carbonate, ammonium carbonate, alkali metal hydrocarbonate and/or ammonium hydrocarbonate in an amount equivalent to 0.01 to 1 g of carbon dioxide, for preparing a transdermal pharmaceutical composition for the treatment of calcification under suppressing the metabolism of acetylsalicylic acid, which is buffered to pH 5.5 to 7.

Taking into account the equilibrium ion household of the organism, it is preferred to use sodium carbonate or sodium hydrocarbonate as alkali metal carbonate or hydrocarbonate.

The transdermal compositions prepared by the use according to the invention may comprise acetylsalicylic acid in amounts usually applied in the known compositions of this type. The compositions prepared by the use according to the invention may contain generally 0.1 to 30% by weight, suitably 0.5 to 20% by weight, preferably 2 to 15% by weight of acetylsalicylic acid, calculated on the total weight of the composition. However, as it is well known to one skilled in pharmacotechnology, the active agent content may also vary with the actual type of the composition.

The compositions prepared by the use according to the invention may comprise the alkali metal carbonate, ammonium carbonate, alkali metal hydrocarbonate and/or ammonium carbonate in an amount equivalent to preferably 0.01 to 0.5 g, more preferably 0.02 to 0.3 g, particularly preferably 0.02 to 0.2 g of carbon dioxide, calculated on 1 g of acetylsalicylic acid.

A further feature of the composition prepared by the use according to the invention is that its pH is of 5.5 to 7, preferably about 6.5. For aqueous compositions, such as emulsions, gels and creams, comprising at least 5% by weight of water, these figures represent the pH measured in the composition itself. For nonaqueous compositions, such as fatty creams or plasters carrying the pharmacons in a dried layer, these figures represent the pH of the composition when contacted with water (e.g. smeared onto wet skin surface or immersed into water). Any of the conventional non-toxic, pharmaceutically acceptable buffers can be used to adjust the pH of the composition to the prescribed value. Of these buffers tertiary amines (such as triethanol amine), salts of tertiary amines and quaternary ammonium salts are preferred.

The compositions prepared by the use according to the invention can be in any form suitable for topical use, such as solutions, emulsions, suspensions, ointments, creams, lotions, shake-up mixtures, gels and plasters. These compositions also comprise carriers, diluents and/or other auxiliary agents conventionally applied in such compositions beside the acetylsalicylic acid active agent, the metabolism delaying agent (alkali metal or ammonium carbonate and/or hydrocarbonate) and the buffer. Examples of such carriers, diluents and other auxiliary agents are as follows: ointment bases, such as vaseline and lanoline; solvents and liquid diluents, such as water, alcohols and glycerol; thickeners and gellifying agents, such as polyvinyl alcohol, polyvinyl acetate and polymeric cellulose derivatives; ionic and nonionic tensides; odourants; substances for adjusting osmotic pressure; colourants and dyestuffs. Preferred representatives of the compositions are those comprising a thickener, optionally along with a stabilizer, since they enable one to apply a relatively high local dose onto the area to be treated. The only specific requirement made on the auxiliary agents is that they should not react with acetylsalicylic acid.

The compositions may optionally also comprise other biologically active substances as activity-complementing agents, examples of which are as follows: antiphlogistic agents, analgesic agents, antirheumatic agents, muscle relaxants, local anaesthetics, pore dilatators and/or skin irritation alleviating agents. The activity-complementing agents also comprise active agents utilized in conventional formulations for the topical treatment of joint and rheumatic pains, such as camphor, menthol, lidocain, diclofenac, snake venom extract, and the like. Preferably the compositions also comprise agents for dilatating skin pores and/or vasopermeability increasing agents as activity-complementing substances, examples of which are capsaicine, histamine and cantharis extract.

If any of the components of the composition comprises alkali metal or ammonium ions, the required amount of alkali metal or ammonium carbonate and/or hydrocarbonate or a part thereof can also be formed from this component by adding a calculated amount of carbon dioxide.

In order to treat calcification, the composition prepared by the use according to the invention is applied onto the body part to be treated. The composition exerts its effect transdermally. If the composition is a nonaqueous one, presence of water should also be provided for e.g. by wetting the area to be treated or by immersing the plaster into water before application.

Further details of the invention are illustrated by the following Examples.

### Example 1

Gels with the compositions given below have been prepared by conventional pharmacotechnological methods:

| | (A) | |
|---|---|---|
| Acetylsalicylic acid | | 5 g |
| Diclofenac-Na | | 4 g |
| Sodium hydrocarbonate | | 3 g |
| Cetyl-trimethyl-ammonium bromide | | 2 g |
| Ethyl-methyl-cellulose | | 5 g |
| Ethanol | | 25 g |
| Distilled water | | 52 g |
| | pH of the gel is 6.1 | |

| | (B) | |
|---|---|---|
| Acetylsalicylic acid | | 10 g |
| Triethanol amine | | 3 g |
| Lidocain | | 3 g |
| Methyl cellulose | | 4 g |
| Ammonium carbonate | | 5 g |
| Ethanol | | 30 g |
| Distilled water | | 45 g |
| | pH of the gel is 6.7. | |

| | (C) | |
|---|---|---|
| Acetylsalicylic acid | | 10 g |
| Sodium carbonate | | 2 g |
| Lidocain | | 4 g |
| Tinctura capsaicini | | 0.5 g |
| Triethanol amine | | 3 g |
| Polyvinyl alcohol | | 5 g |
| Ethanol | | 22 g |
| Distilled water | | 53.5 g |
| | pH of the gel is 6.5. | |

### Example 2

### Biological activity tests and their results

### (A) Examination of the metabolism of acetylsalicylic acid

The tests were performed on 6 weeks old male New-Zealand giant white rabbits. Before testing the animals were subjected to radiography to ascertain that they do not suffer from calcification. The hair was shaved off from the backs of the animals, and 0.6 g of a gel with the following composition was applied onto the skin:

| | |
|---|---|
| acetylsalicylic acid | 0.05 g |
| ethanol | 0.05 g |
| methyl cellulose | 0.02 g |
| distilled water | 0.48 g |

Blood samples were taken periodically, as given in Table 1, from the jugular veins of the animals, the samples were centrifuged to separate the plasm, and the acetylsalicylic acid (ASA) and salicylic acid (SA) contents of the samples were measured by HPLC. The centrifuged plasms were stored at -22°C until analysis in order to avoid the occurrence of any ex vivo metabolism upon the effect of esterases present in the plasm.

The results are summarized in Table 1.

**Table 1**

| Salicylic acid and acetylsalicylic acid contents of blood samaples, µg/ml of plasm | | | | | | | | |
|---|---|---|---|---|---|---|---|---|
| Time hours | Rabbit 1 | | Rabbit 2 | | Rabbit 3 | | Rabbit 4 | |
| | ASA | SA | ASA | SA | ASA | SA | ASA | SA |
| 0 | 0 | BLD | 0 | BLD | 0 | BLD | 0 | BLD |
| 0.25 | 0.088 | 18.74 | BLD | 17.07 | 0.086 | 25.78 | 0.312 | 45.29 |
| 0.5 | 0.094 | 29.02 | 0.105 | 28.48 | 0.091 | 28.767 | 0.192 | 67.74 |
| 0.75 | 0.115 | 40.81 | BLD | 44.02 | 0.108 | 39.969 | 0.19 | 57.49 |
| 1 | 0.098 | 29.07 | 0.108 | 51.85 | 0.134 | 60.132 | 0.132 | 109.11 |
| 1.25 | 0.084 | 41.7 | 0.094 | 47.98 | 0.128 | 48.574 | 1.238 | 98.04 |
| 1.5 | 0.081 | 28.59 | 0.096 | 50.87 | BLD | 56.27 | 0.201 | 94.31 |
| 1.75 | 0.076 | 35.15 | BLD | 77.06 | BLD | 52.076 | 0.136 | 98.4 |
| 2 | BLD | 37 | BLD | 60.84 | BLD | 69.485 | 0.163 | 119.52 |

| | | | | | | | | |
|---|---|---|---|---|---|---|---|---|
| BLD = below the limit of detection | | | | | | | | |

The data of Table 1 clearly demonstrate that the salicylic acid content of the blood samples was always at least the hundredfold of the acetylsalicylic acid content. The results of the meassurements performed in every 15 minutes verify that acetylsalicylic acid metabolises in the living organism within 15 minutes with the formation of salicylic acid and acetic acid.

The above test was repeated on the same animals with the difference that 0.01 g of sodium carbonate was also added to the gel and the gel was buffered to pH 6.5. The results are listed in Table 2.

**Table 2**

| Salicylic acid and acetylsalicylic acid contents of blood samples, µg/ml of plasm | | | | | | | | |
|---|---|---|---|---|---|---|---|---|
| Time hour | Rabbit 1 | | Rabbit 2 | | Rabbit 3 | | Rabbit 4 | |
| | ASA | SA | ASA | SA | ASA | SA | ASA | SA |
| 0 | 0 | 0 | 0 | 0 | 0 | 0 | 0 | 0 |
| 0.25 | 0.57 | 0.9 | 0.67 | 0.95 | 0.6 | 0.86 | 0.81 | 0.8 |
| 0.5 | 5.24 | 0.98 | 5.91 | 0.99 | 6.14 | 0.96 | 6.45 | 0.97 |
| 0.75 | 7.9 | 2.1 | 8.84 | 1.97 | 9.44 | 2.81 | 9.68 | 2.54 |
| 1 | 7.7 | 3.02 | 8.84 | 2.25 | 9.21 | 2.94 | 8.89 | 2.89 |
| 1.5 | 7.12 | 2.69 | 8.84 | 2.59 | 8.94 | 2.86 | 8.98 | 3.08 |
| 2 | 6.98 | 3.11 | 8.14 | 2.87 | 8.56 | 3.12 | 8.58 | 2.98 |
| 3 | 5.65 | 7.98 | 6.47 | 6.57 | 7.03 | 7.88 | 7.14 | 7.43 |
| 4 | 4.55 | 12.24 | 5.14 | 15.4 | 5.71 | 13.57 | 5.47 | 12.21 |

From the data of Table 2 it appears that the amount of acetylsalicylic acid present in the blood of the animals stabilized after a short induction period. An extreme slow down in the metabolism of acetylsalicylic acid could be observed. Metabolism started essentially at about the third-fourth hour, however, 25 % of acetylsalicylic acid remained unmetabolised even after 4 hours. It should be stressed here that this test was performed on healthy animals, where no dissolution of calcification and no formation of calcium acetylsalicylate due to this dissolution proceeds. Taking into account that acetylsalicylic acid once reacted with the calcified area and thus converted to calcium acetylsalicylate can no more be metabolised but is excreted from the organism in unchanged form (which was checked by urine analyses performed on rabbits kept on specific calcium-rich diet), undesired calcium acetate formation can safely be avoided when a calcified organ is treated with the composition according to the invention.

### (B) Examination of calcification-dissolving effects under ex vivo conditions

The tests were performed on intact pig joints. One marble chip, about 5 mm in diameter and about 1-1.5 g in weight, was placed onto each of the joints, and the chips were smeared with a gel of the composition as given in point (A) of Example 1. After 8 hours this treatment was repeated, and then the joints were allowed to stand overnight. Next day the effects were assessed visually. No marble residue could be observed on any of the joints. Upon subjecting the joints to surgical and radiographic examination no damage of the bone, of the synovial membrane or of the medullary substance could be detected, either.

## Claims

1. Use of acetylsalicylic acid together with a carrier, diluent and/or other auxiliary agent suitable for transdermal application, and for 1 g of acetylsalicylic acid, an alkali metal carbonate, ammonium carbonate, alkali metal hydrocarbonate and/or ammonium hydrocarbonate in an amount equivalent to 0.01 to 1 g of carbon dioxide, for preparing a transdermal pharmaceutical composition for the treatment of calcification under suppressing the metabolism of acetylsalicylic acid, which is buffered to pH 5.5 to 7.

2. Use according to claim 1, in which 0.1 to 30% by weight of acetylsalicylic acid, calculated on the total weight of the composition, is employed.

3. Use according to claim 1 or 2, in which for 1 g of acetylsalicylic acid an alkali metal carbonate, ammonium carbonate, alkali metal hydrocarbonate and/or ammonium hydrocarbonate in an amount equivalent to 0.01 to 0.5 g of carbon dioxide is employed.

4. Use according to claim 3, in which for 1 g of acetylsalicylic acid an alkali metal carbonate, ammonium carbonate, alkali metal hydrocarbonate and/or ammonium hydrocarbonate in an amount equivalent to 0.02 to 0.3 g of carbon dioxide is employed.

5. Use according to claim 4, in which for 1 g of acetylsalicylic acid an alkali metal carbonate, ammonium carbonate, alkali metal hydrocarbonate and/or ammonium hydrocarbonate in an amount equivalent to 0.02 to 0.2 g of carbon dioxide is employed.

6. Use according to any of claims 1 to 5, in which sodium carbonate as alkali metal carbonate and sodium hydrocarbonate as alkali metal hydrocarbonate is employed.

7. Use according to any of claims 1 to 6, in which as buffer for adjusting the pH to 5.5 to 7 a tertiary amine, a tertiary amine salt or a quaternary ammonium salt is employed.

8. Use according to claim 7, in which triethanol amine is employed as buffer.

9. Use according to any of claims 1 to 8, in which also one or more antiphlogistic agent(s), analgesic agent(s), antirheumatic agent(s), muscle relaxant(s), local anaesthetic(s), pore dilatator(s) and/or skin irritation alleviating agent(s) is/are employed as activity-complementing agent(s).

10. A transdermal pharmaceutical composition comprising acetylsalicylic acid together with a carrier, diluent and/or other auxiliary agent suitable for transdermal application, wherein the composition also comprises for 1 g of acetylsalicylic acid, an alkali metal carbonate, ammonium carbonate, an alkali metal hydrocarbonate and/or ammonium hydrocarbonate in an amount equivalent to 0.01 to 1 g of carbon dioxide, and is buffered to pH 5.5 to 7, **characterized in that** it comprises as buffer for adjusting the pH to 5.5 to 7 a tertiary amine, a tertiary amine salt or a quaternary ammonium salt.

11. A composition as claimed in claim 10, which comprises triethanol amine as buffer.

## Patentansprüche

1. Verwendung von Acetylsalicylsäure zusammen mit einem Träger, Verdünnungsmittel und/oder einem anderen Hilfsmittel, das für die transdermale Applizierung geeignet ist, und für 1 g Acetylsalicylsäure eines Alkalimetallcarbonats, Ammoniumcarbonats, Alkalimetallhydrogencarbonats und/oder Ammoniumhydrogencarbonats in einer Menge, die zu 0,01 bis 1 g Kohlendioxid äquivalent ist, zur Herstellung einer transdermalen pharmazeutischen Zusammensetzung für die Behandlung von Kalzifikation unter Unterdrückung des Stoffwechsels von Acetylsalicylsäure, welche auf einen pH-Wert von 5,5 bis 7 gepuffert ist.

2. Verwendung gemäß Anspruch 1, bei der 0,1 bis 30 Gew.-% an Acetylsalicylsäure, berechnet bezüglich des Gesamtgewichtes der Zusammensetzung, angewendet werden.

3. Verwendung gemäß Anspruch 1 oder 2, bei der für 1 g Acetylsalicylsäure ein Alkalimetallcarbonat, Ammoniumcarbonat, Alkalimetallhydrogencarbonat und/oder Ammoniumhydrogencarbonat in einer Menge verwendet wird, die zu 0,01 bis 0,5 g an Kohlendioxid äquivalent ist.

4. Verwendung gemäß Anspruch 3, bei der für 1 g Acetylsalicylsäure ein Alkalimetallcarbonat, Ammoniumcarbonat, Alkalimetallhydrogencarbonat und/oder Ammoniumhydrogencarbonat in einer Menge verwendet wird, die zu 0,02 bis 0,3 g an Kohlendioxid äquivalent ist.

5. Verwendung gemäß Anspruch 4, bei der für 1 g Acetylsalicylsäure ein Alkalimetallcarbonat, Ammoniumcarbonat, Alkalimetallhydrogencarbonat und/oder Ammoniumhydrogencarbonat in einer Menge verwendet wird, die zu 0,02 bis 0,2 g an Kohlendioxid äquivalent ist.

6. Verwendung gemäß mindestens einem der Ansprüche 1 bis 5, bei der Natriumcarbonat als Alkalimetallcarbonat und Natriumhydrogencarbonat als Alkalimetallhydrogencarbonat angewendet werden.

7. Verwendung gemäß mindestens einem der Ansprüche 1 bis 6, bei der als Puffer zur Einstellung des pH-Wertes auf 5,5 bis 7 ein tertiäres Amin, ein tertiäres Aminsalz oder ein quaternäres Ammoniumsalz angewendet wird.

8. Verwendung gemäß Anspruch 7, bei der Triethanolamin als Puffer angewendet wird.

9. Verwendung gemäß mindestens einem der Ansprüche 1 bis 8, bei der ebenfalls ein oder mehrere entzündungshemmende(s) Mittel, analgetische(s) Mittel, antirheumatische(s) Mittel, Muskelrelaxant(ein), Lokalanestetika(um), Porendilator(en) und/oder die Hautreizung lindernde(s)Mittel als die Aktivität komplementierende(s) Mittel zur Anwendung kommt/kommen.

10. Transdermale pharmazeutische Zusammensetzung, umfassend Acetylsalicylsäure zusammen mit einem Träger, Verdünnungsmittel und/oder einem anderen Hilfsmittel, das für die transdermale Applizierung geeignet ist, wobei die Zusammensetzung ebenfalls für 1 g Acetylsalicylsäure ein Alkalimetallcarbonat, Ammoniumcarbonat, ein Alkalimetallhydrogencarbonat und/oder Ammoniumhydrogencarbonat in einer Menge umfasst, die 0,01 bis 1 g Kohlendioxid äquivalent ist, und auf einen pH-Wert von 5,5 bis 7 gepuffert ist, **dadurch gekennzeichnet, dass** sie als Puffer zur Einstellung des pH-Wertes auf 5,5 bis 7 ein tertiäres Amin, ein tertiäres Aminsalz oder ein quaternäres Ammoniumsalz umfasst.

11. Zusammensetzung gemäß Anspruch 10, welche Triethanolamin als Puffer umfasst.

## Revendications

1. Utilisation d'acide acétylsalicylique conjointement avec un support, un diluant et/ou un autre agent auxiliaire approprié pour une application transdermique, et pour 1 g d'acide acétylsalicylique, un carbonate de métal alcalin, du carbonate d'ammonium, un hydrocarbonate de métal alcalin et/ou de l'hydrocarbonate d'ammonium dans une quantité équivalente à 0,01 à 1 g de dioxyde de carbone, pour préparer une composition pharmaceutique transdermique destinée au traitement de la calcification sous suppression du métabolisme de l'acide acétylsalicylique, qui est tamponnée à pH 5,5 à 7.

2. Utilisation selon la revendication 1, dans laquelle 0,1 à 30 % en poids d'acide acétylsalicylique, calculés sur le poids total de la composition, sont employés.

3. Utilisation selon la revendication 1 ou 2, dans laquelle pour 1 g d'acide acétylsalicylique, un carbonate de métal alcalin, de carbonate d'ammonium, un hydrocarbonate de métal alcalin et/ou de l'hydrocarbonate d'ammonium dans une quantité équivalente à 0,01 à 0,5 g de dioxyde de carbone est employé.

4. Utilisation selon la revendication 3, dans laquelle pour 1 g d'acide acétylsalicylique, un carbonate de métal alcalin, du carbonate d'ammonium, un hydrocarbonate de métal alcalin et/ou de l'hydrocarbonate d'ammonium dans une quantité équivalente à 0,02 à 0,3 g de dioxyde de carbone est employé.

5. Utilisation selon la revendication 4, dans laquelle pour 1 g d'acide acétylsalicylique, un carbonate de métal alcalin, du carbonate d'ammonium, un hydrocarbonate de métal alcalin et/ou de l'hydrocarbonate d'ammonium dans une quantité équivalente à 0,02 à 0,2 g de dioxyde de carbone est employé.

6. Utilisation selon l'une quelconque des revendications 1 à 5, dans laquelle du carbonate de sodium en tant que carbonate de métal alcalin et de l'hydrocarbonate de sodium en tant qu'hydrocarbonate de métal alcalin est employé.

7. Utilisation selon l'une quelconque des revendications 1 à 6, dans laquelle comme tampon pour ajuster le pH à 5,5 à 7, une amine tertiaire, un sel d'amine tertiaire ou un sel d'ammonium quaternaire est employé.

8. Utilisation selon la revendication 7, dans laquelle de la triéthanolamine est employée en tant que tampon.

9. Utilisation selon l'une quelconque des revendications 1 à 8, dans laquelle également un ou plusieurs agent(s) antiphlogistique(s), agent(s) analgésique(s), agent(s) antirhumatismal/antirhumatismaux, relaxant(s) musculaire(s), anesthésique(s) local/locaux, dilatateur(s) des pores et/ou agent(s) soulageant une irritation de la peau est/sont employé(s) en tant qu'agent(s) de complémentation de l'activité.

10. Composition pharmaceutique transdermique comprenant de l'acide acétylsalicylique conjointement avec un support, un diluant et/ou un autre agent auxiliaire approprié pour une application transdermique, où la composition comprend également pour 1 g d'acide acétylsalicylique, un carbonate de métal alcalin, du carbonate d'ammonium, un hydrocarbonate de métal alcalin et/ou de l'hydrocarbonate d'ammonium dans une quantité équivalente à 0,01 à 1 g de dioxyde de carbone, et est tamponnée à pH 5,5 à 7, **caractérisée en ce qu'**elle comprend comme tampon pour ajuster le pH à 5,5 à 7, une amine tertiaire, un sel d'amine tertiaire ou un sel d'ammonium quaternaire.

11. Composition selon la revendication 10, qui comprend de la triéthanolamine en tant que tampon.
